# EUROPEAN PATENT APPLICATION

(11) **EP 2 236 077 A1**
(43) Date of publication of application: **06.10.2010**
(21) Application number: 09156930.1
(22) Date of filing: 31.03.2009
(51) Int. Cl.: A61B 5/00, A61B 5/15, G01N 11/16

(54) **Medical device for measuring an analyte concentration**

(71) Applicant: Sensile Pat AG, 4614 Haegendorf (CH)
(72) Inventor: Beyer, Uwe, 4600 Olten (CH); Robin, Franck, 5600 Lenzburg (CH); Straessler, Sigfrid, 1113 St-Saphorin-sur-Morges (CH)
(74) Representative: Reuteler, Raymond Werner

(57) **Abstract**

There is provided a medical device for measuring an analyte concentration in the subcutaneous tissue of a patient, the medical device comprising a body access unit and a processing unit. The body access unit and the processing unit are functionally connected when an analyte concentration is measured. The body access unit comprises a transcutaneous dialysis member for accessing the body of a patient. A fluid reservoir is contained at least partially in the transcutaneous dialysis member, and the fluid reservoir is at least partially bounded by a porous membrane. The fluid reservoir contains an analyte sensitive liquid. The processing unit comprises an excitation means adapted to act on a displacement member of the body access unit to generate a flow of the analyte sensitive liquid in the fluid reservoir. The processing unit further comprises a displacement sensor adapted to measure a displacement behavior of the displacement means, a damping of the displacement behavior caused by at least the viscosity of the analyte sensitive liquid contained in the fluid reservoir.

## Description

The present invention relates to a medical device suitable for measuring an analyte concentration.

The regular or continuous measurement of an analyte concentration is necessary in the control or therapy of many conditions, such as diabetes. For instance, diabetic patients may require measurement of their blood glucose level several times a day, in order to adapt the administration of insulin accordingly. More measurements of the blood glucose level allow for drug administration regimes which regulate the blood glucose level of the diabetic patient more efficiently, i.e. the fluctuations of the blood glucose level may be kept within a physiological range. Hence, it is crucial for a successful treatment of diabetic patients to obtain accurate, undelayed, and continuous information about the blood glucose level.

Various different medical devices have been proposed for the monitoring of blood glucose levels. Most conventional blood glucose monitoring devices make use of test strips which work on electro-chemical principles, whereby the patient withdraws a droplet of blood for each measurement, requiring uncomfortable finger pricking methods. In order to avoid the pain caused by finger pricking and to allow more frequent, or continuous, control of glycaemia a variety of implantable sensors, including transdermal or subcutaneous sensors, are being developed for continuously detecting and/or quantifying blood glucose values. Glucose sensors for frequent or continuous glucose monitoring based on electrochemical, affinity, or optical sensors have been widely investigated.

One type of affinity sensor uses affinity viscosimetry, whereby a sensitive polymeric solution, which changes its viscosity when the concentration of the analyte changes, is used. Some sensitive solutions include dextrane molecules that are cross-linked by Concanavalin A, a tetravalent affinity receptor with affinity to the glucose end-groups of the dextrane molecules and the analyte glucose. Increasing the concentration of the free analyte, the viscosity of the solution decreases strongly. This allows measuring very accurately the analyte concentration, because the affinity binding may be very specific to the analyte. Moreover, the analyte is not consumed as is the case in electrochemical sensors.

Of the known affinity viscosimetry sensors in the prior art, two types may be distinguished: implantable sensors and invasive or minimally-invasive sensors.

An example of an implantable analyte sensor utilizing affinity viscosimetry for measuring analyte concentration is described in German patent application DE 195 01 159 A1. Disclosed therein is an implantable sensor, comprising a dialysis chamber filled with a glucose sensitive polymer solution and another fluid such as silicon oil, the two fluids being adjacent to each other and forming a stable boundary layer. The implantable sensor further comprises a metallic membrane for oscillating the silicon oil, electrodes connected by coaxial transmission lines to a communication unit, and an electric coil. Outside of the body there is provided a display- and evaluation unit, also equipped with an electric coil for communication with the implantable sensor.

US 2001 045 122 A1 discloses an implantable sensor containing electronic components, a glucose sensitive polymer solution, and a dialysis chamber. Viscosity is measured by moving a flexible member relative to a rigid member within the dialysis chamber, and measuring the return behavior of the flexible member back to its initial position. Again, the signal evaluation circuit is located outside the body and stays in communication with the electronic components of the implantable sensor.

Another example of an implantable glucose sensor utilizing affinity viscosimetry for measuring glucose concentration is disclosed in PCT application WO 2004 037 079 A1. The implantable sensor is for prolonged implantation within the body of the patient and comprises a dialysis chamber filled with a glucose sensitive polymer solution and a rotating or oscillating measurement organ positioned in the solution. A decay behavior of the excited measurement organ is used to determine viscosity of the glucose sensitive polymer solution. A user device located outside the body controls and evaluates the measurement.

WO 2008/102001 discloses a viscosimetric affinity sensor comprising a dialysis chamber having a glucose permeable membrane and containing a glucose sensitive liquid, the sensor further comprising a restrictive passage in the chamber, the chamber being closed at one end thereof by a membrane. An actuator that generates pressure within the chamber causing the sensitive liquid to flow through the restrictive passage, the flow of which is dependent on the viscosity of the sensitive liquid and therefore on the glucose concentration, influences the displacement of the external membrane. Measuring the displacement of the external membrane thus provides a measure of the viscosity of the sensitive liquid and hence the glucose concentration.

A drawback of certain implantable viscosimetric affinity sensors disclosed in the prior art, is that they measure an absolute change in the viscosity of the liquid, which is strongly dependent on temperature and other factors. For accurate absolute glucose measurement it is important to establish a reference value that allows compensating changes in viscosity due to the influencing factors.

In WO 2008/102001, it is proposed to provide a second chamber with a sensitive liquid in order to provide a reference measurement to calibrate for variations in viscosity due to temperature or other factors. It however requires a second implantable member and thus increases invasiveness and moreover other changes in the sensitive liquids in the separate chamber may occur that lead to an offset which is not accounted for.

US2001035047A1 and US2003054560A1 disclose affinity sensors for glucose concentration determination using a reference measurement to calculate a relative fluidity to reduce the dependency of the measurement on temperature variations. Drawbacks of these sensors are the complexity of the disposable part, the size of the needle required and the use of a single-pass reservoir and waste which limit the application duration

Long term implantable sensors pose a number of challenges. It is difficult to prevent encapsulation by body tissue in long term implantation and long term stability of the glucose sensitive material must be guaranteed for the duration the sensor is implanted. It is also important to ensure stability of the dialysis membrane and to prevent clogging through particles contained in the body fluids. One also needs to ensure reliable communication between the implantable sensor and the external controller device and the sensor must be supplied with power over the time the sensor remains implanted.

In German patent application DE 197 14 087 A1 a minimally-invasive viscosimetric affinity sensor is disclosed. The sensor comprises a flow path with a dialysis chamber section and a measurement chamber section, whereas the dialysis chamber section is contained in a needle and the measurement chamber section is located downstream of the needle. Accordingly, new analyte sensitive liquid flows continuously from a reservoir through the dialysis chamber section and then through the measurement chamber section where the viscosity is determined.

The aforementioned system requires a fluid pump, a reservoir with the analyte sensitive liquid, a complex and possibly bulky dialysis needle, a sensor chamber section, and a waste reservoir for the used analyte sensitive liquid, and therefore miniaturization of this system is limited to the size of the above-mentioned components. Accordingly, a patch-like device containing all above-mentioned components is not convenient to carry next to the patient's skin for daily use.

There is an ongoing need to provide medical devices for measuring an analyte concentration that are convenient to use by patients and that enable users to continuously measure analyte concentration over a period of several days.

An object of this invention is to provide an analyte concentration measurement system that is convenient to use and that enables frequent, accurate and reliable analyte concentration measurement.

It would be advantageous to provide an analyte concentration measurement system that is easy to implement.

It would be advantageous to provide an analyte concentration measurement system that offers rapid analyte measurement.

It would be advantageous to provide an analyte concentration measurement system that is compact, light weight and economical to manufacture.

It would be advantageous to provide an analyte concentration measurement system that is economical to use in long term therapies.

It would be advantageous to provide an analyte concentration measurement system that is convenient and easy to wear and to manipulate.

It would be advantageous to provide an analyte concentration measurement system that can be fully encapsulated and is water-proof.

Objects of this invention have been achieved by providing an analyte concentration measurement system according to claim 1, and a method of operating an analyte concentration measurement system according to claim 16.

Disclosed herein is an analyte measurement system comprising a body access unit, the body access unit including a transcutaneous or implantable dialysis member comprising an analyte porous membrane, a fluid reservoir extending at least partially within a cavity of the dialysis member, the fluid reservoir comprising a analyte exchange section bounded by at least said analyte porous membrane, an analyte sensitive liquid contained in the fluid reservoir, and a displacement member adapted to be displaced in a predefined manner, the displacement of the displacement member inducing flow of the analyte sensitive liquid in the fluid reservoir, wherein the displacement member comprises an extension insertable in the analyte exchange section and configured to displace analyte sensitive liquid in and out of the analyte exchange section.

The analyte measurement system may further include a separate processing unit, whereas the body access unit and the processing unit are physically independent of each other but configured to be placed in close contact to each other when an analyte concentration is to be measured. The body access unit comprises a transcutaneous or implantable dialysis member for accessing the body of a patient. A fluid reservoir extends at least partially in a cavity of the dialysis member, the cavity being bounded by an analyte porous membrane. The fluid reservoir contains an analyte sensitive liquid.

Coupling means may be provided on the body access unit and on the processing unit for mechanically securing the processing unit to the body access unit in a situation of use. The processing unit comprises an excitation means configured to act on the displacement member of the body access unit to generate a flow of the analyte sensitive liquid in the fluid reservoir. The processing unit further comprises a displacement sensor adapted to measure a displacement behavior of the displacement member in the body access unit.

The resistance to the movement of the displacement member caused by the analyte sensitive fluid surrounding the displacement member is dependent on the viscosity of the fluid. The displacement behavior of the displacement member is affected at least partially by the viscosity of the analyte sensitive fluid in the cavity of the dialysis member and surrounding the displacement member extension inserted in the cavity.

A method of operating the analyte concentration measurement device according to the invention includes the steps of: actuating the excitation means to displace the displacement member, thereby displacing analyte sensitive liquid in and out of the cavity of the analyte exchange section of the dialysis member with the displacement member extension; and measuring the viscosity of the analyte sensitive liquid based on the displacement behavior of the displacement member.

A method of measuring an analyte concentration according to the invention comprises the steps of: i) providing a medical device comprising: a body access unit and a processing unit, the body access unit comprising a transcutaneous dialysis member having a glucose exchange section with an analyte porous membrane, a fluid reservoir containing an analyte sensitive liquid, and a displacement member comprising an extension inserted at least partially in a cavity of the transcutaneous dialysis member, and the processing unit comprising an excitation means configured to displace the displacement means; ii) displacing the displacement member by the excitation means, thereby displacing analyte sensitive liquid in and out of the cavity of the dialysis member with the displacement member extension; iii) measuring the displacement behavior of the displacement member; and iv) determining an analyte concentration correlated to the displacement behavior of the displacement member.

The movement of the displacement member may comprise a translational movement such that the extension is inserted further into the cavity of the transcutaneous dialysis member thus displacing analyte sensitive liquid out of said cavity into the fluid reservoir, and a return translational movement retracting the extension such that analyte sensitive fluid from the fluid reservoir enters the cavity. The actuation of the displacement member in this variant is an oscillation or oscillatory displacement. It should be noted that the terms "oscillation" or "oscillatory displacement" are meant herein to encompass a displacement that may have more than one cycle or that could be less than a full oscillation cycle, for example the displacement member may be driven in only one direction and then released, whereby the return displacement behavior of the displacement member into its original position is measured. The oscillatory behavior of the displacement member depends on the dimensions of the components on the one hand, and on the damping caused by the analyte sensitive liquid in the fluid reservoir and in the cavity of the transcutaneous dialysis member on the other hand. The damping effect of the analyte sensitive liquid depends inter alia on the viscosity of the analyte sensitive liquid, which, in the transcutaneous dialysis member, varies with the concentration of analyte.

In an alternative variant, the extension of the displacement member may comprise blades or a helical thread or equivalent fluid pumping means therealong and the movement of the displacement member may comprise a rotational movement such that as the extension rotates inside the cavity of the transcutaneous dialysis member, analyte sensitive liquid is circulated out of said cavity into the fluid reservoir and from the fluid reservoir into the cavity.

In a further alternative embodiment, the displacement member may be configured to move both in a translational movement and a rotational movement in order to pump fluid out of, respectively into the cavity of the dialysis member, and to mix the fluid in the fluid reservoir, at least in the vicinity of the connection between the cavity of the dialysis member and the fluid reservoir.

In the initial displacement of the displacement member into the cavity of the dialysis member, the analyte sensitive fluid expelled from the cavity has a viscosity that is dependant on the concentration of analyte in the exchange section of the cavity (hereinafter referred to as the analyte infused sensitive fluid), which is dependant on the concentration of analyte in the external fluid (e.g. interstitial body fluid or blood) surrounding the dialysis member in view of the exchange of analyte molecules through the analyte porous membrane. The flow of analyte sensitive fluid expelled from the dialysis member cavity is restricted by the resistance acting on the dialysis member extension inserted in the cavity that is dependant on the viscosity of the fluid and the geometry between the cavity and the extension of the displacement member. After equilibration of the analyte concentration within the dialysis cavity and outside during dialysis time between different measuring cycles the displacement behavior of the displacement member thus depends on the fluid flow resistance acting on the displacement member which in turn depends at least partially on the viscosity of the analyte sensitive liquid flowing in and out of the dialysis member cavity. During the initial movement of the displacement member, the fluid in the exchange section of the dialysis member cavity has an analyte concentration that corresponds to the analyte concentration on the outer side of the porous membrane and thus has a viscosity correlated to the external analyte concentration. After the initial movement expelling fluid from the cavity of the dialysis member and subsequent refilling of the cavity section with fresh fluid from the reservoir and repeating the refill process within a few seconds for few times, the fluid in the cavity section of the dialysis member has a viscosity corresponding essentially to the viscosity of the analyte sensitive fluid in the reservoir that is not correlated to the external analyte concentration and thus serves as a reference.

The behavior of the displacement of the displacement member in the initial movement or movement cycle can be compared to the displacement behavior of the displacement member in one or more subsequent movements or movement cycles thereby allowing calibration of the viscosity of the analyte infused sensitive fluid with the viscosity of the reference sensitive fluid in the fluid reservoir.

As described in in Beyer, U., Ehwald, R., 2000, Biotechnology Progress 16, 1119-1123, this relative viscosity measurement (relative fluidity being the reciprocal value, respectively) eliminates or reduces the requirements for regular calibration of the medical device measurement parameters by external calibrating means, as found in systems based on measuring an absolute viscosity. Thus, the relative increase in viscosity is well correlated to the absolute analyte concentration in the analyte infused sensitive fluid and is little affected by the temperature or ageing of the sensitive fluid.

The modular set-up of the medical device according to this invention for measuring an analyte concentration allows the body access unit to be disposable and the processing unit to be reusable. "Disposable" shall mean that this part is normally discarded after one application, i.e. after the body access unit is withdrawn from the patient's body or after several days of use. Typically, the body access unit contains sterile parts, and the time period of use for the body access unit lasts from hours to weeks. "Reusable" shall mean that this part is normally repeatedly used with several disposable units. The reusable unit does not contain sterile parts, and is typically repeatedly used, from weeks to years.

Advantageously, the reusable unit contains valuable elements, such as electronics, sensors, or wireless communication modules, whereas the disposable unit may contain less valuable elements, such as a needle, small amounts of analyte sensitive liquid, and membranes. Therefore, a medical device for measuring an analyte concentration according to this invention is economically and ecologically advantageous, because only consumable parts most preferably without electronic components may be discarded regularly, whereas valuable or reusable parts can be reused over a prolonged period of time.

There is provided a method according to which one processing unit is repeatedly used with at least two body access units. The one processing unit may be used for approximately two to four years, whereas one body access unit may be used for approximately three to ten days.

The functional interface between the body access unit and the processing unit may be a wireless electromagnetic connection between the excitation means contained in the processing unit and the displacement member contained in the body access unit. This interface is advantageously adapted to allow repeated attachment and detachment of the processing unit to the body access unit, in order to provide flexibility and freedom when using this system in daily life.

The term "body access unit" is intended to include any type of medical device or parts thereof to be carried in or on a patient for measuring analyte concentration into or traversing the skin, including subcutaneous, intradermal, intra-peritoneal, intravenous, spinal, intra-articular, invasive, semi-invasive, minimally-invasive, and intra-dermal.

The body access unit is adapted for transdermal application. This may be achieved by providing a rigid needle, or alternatively by using a rigid support structure when inserting the transcutaneous dialysis member into the body of a patient. The body access unit further may be adapted to adhere to the skin of a patient, employing a support member in the form of a patch or other suitable attaching means. When properly attached to the skin, the transcutaneous dialysis member of the body access unit is less likely to injure the patient. Additionally, the body access unit may be left on the body of the patient, while the processing unit is detached. This offers flexibility in use and allows the patient to preserve the processing unit when taking a shower, bathing, or otherwise exposing the medical device to external hazards.

For physical connection to the processing unit, coupling means may be provided on the body access unit. For this purpose, mechanical or magnetic coupling means may be provided, whereas a male part and a female part are each located on one of the units respectively. Advantageously, the coupling means are configured such that the two units are repeatedly attachable and detachable. Advantageously, no electrical coupling means are required between the body access unit and the processing unit thanks to the wireless electromagnetic connection. It is thus possible to fully seal and make the processing unit waterproof.

The porous membrane of the dialysis member advantageously features pores with a diameter that allows analytes, such as glucose, to pass, and at the same time prohibits larger molecules, such as proteins contained in the analyte sensitive liquid or in the body fluids, from passing through the porous membrane.

The analyte sensitive liquid consists preferably of a polymer solution. In the case that the analyte to be measured is glucose, a solution containing concanavalin A and dextran or phenylboronic acids may advantageously be utilized.

The term "processing unit" is intended to include any type of medical device or parts thereof for measuring analyte concentration adapted to carry out at least one step in the process of measuring an analyte concentration. For instance, the processing unit may be made of one single part, or comprising multiple sub-units.

Advantageously, the processing unit comprises an excitation means adapted to transmit energy from the processing unit onto the displacement member of the body access unit in order to move the displacement member. The excitation means may for example comprise a magnet or electromagnet formed by a coil, that generates a magnetic field acting on a magnet of the displacement member. In a variant the excitation can be provided via a mechanical interface from the processing unit to the body access unit.

The processing unit comprises a displacement sensor adapted to measure the displacement behavior of the displacement member which is affected by the damping of the analyte sensitive liquid flow contained in the transcutaneous dialysis member which is dependant, at least in part, on the viscosity of an analyte sensitive liquid. The displacement sensor may be any kind of sensor suitable for directly or indirectly detecting the displacement of the displacement member in the body access unit, e.g. a Hall sensor, a capacitive sensor, or an optical sensor such as a laser sensor. The sensor may also be integrated in the regulation loop of the excitation means in the processing unit. For example, the displacement behavior sensor may include a force sensing function integrated in the control circuit of the electromagnetic drive of the excitation means.

The processing unit may advantageously further comprise a communication member adapted to communicate with a user interface device. The communication between the processing unit and the user interface device is preferably achieved by wireless communication, but alternatively may also include cable communication. The user interface device may be a separate device. Alternatively, a cell phone, a wristwatch, a PDA, or other electronic user devices may be employed. The user interface device is preferably adapted to display information obtained by the medical device for measuring analyte concentration. Furthermore the user interface device may serve as an interface to connect to a personal computer, or to manage the medical device for measuring an analyte concentration.

In an alternative embodiment, the processing unit itself is capable of connecting to a personal computer, and of managing the measurement of an analyte concentration. The processing unit may comprise user input components, e.g. buttons, and or user output components for displaying information related to the measurement of an analyte concentration.

The processing unit may further comprise an alarm unit adapted to warn a user if a measured value of the analyte concentration is outside a predetermined range. This is advantageous e.g. in the treatment of diabetic patients, as the patient may be warned in case the glucose concentration measured is not within a physiologically healthy range. Besides setting off an alarm, the measured analyte concentration may be transferred to a medicament delivery device by means of a control unit, in order to regulate the analyte concentration in the patient in a closed loop fashion, e.g. an insulin delivery device for patients with diabetes.

Further objects and advantageous aspects of the invention will be apparent from the claims and the following detailed description of preferred embodiments of the invention in conjunction with the drawings in which:
Fig 1 shows a cross-sectional view of an embodiment of an analyte concentration measurement system according to the invention;
Fig 2 shows a cross-sectional view of an embodiment of an analyte concentration measurement system according to the invention with body access unit and processing unit separated;
Fig 3a shows a detailed view of a first embodiment of a transcutaneous dialysis member of an analyte concentration measurement system according to the invention;
Fig 3b shows a detailed view of a second embodiment of a transcutaneous dialysis member of an analyte concentration measurement system according to the invention;
Fig 3c shows a detailed view of a third embodiment of a transcutaneous dialysis member of an analyte concentration measurement system according to the invention;
Fig 4a shows a detailed view of an analyte exchange section and an analyte measurement section of a transcutaneous dialysis member of an analyte concentration measurement system according to the invention;
Fig 4b shows a detailed view of another embodiment of an analyte exchange section and an analyte measurement section of a transcutaneous dialysis member of an analyte concentration measurement system according to the invention;
Fig 5a shows a detailed view of an upper part of a displacement member extension inserted in a dialysis member cavity of an analyte concentration measurement system according to the invention;
Fig 5b shows a detailed view of another embodiment of an upper part of a displacement member extension inserted in a dialysis member cavity of an analyte concentration measurement system according to the invention;
Fig 6a shows a simplified cross section of an embodiment of a body access unit of an analyte concentration measurement system according to the invention, with a displacement member in a retracted position;
Fig 6b is a view similar to figure 6a except that the displacement member is in a fully inserted position;
Fig 6c is a view similar to figures 6a and 6b except that the displacement member is in an intermediate position;
Fig 7a shows a simplified cross section of another embodiment of a body access unit of an analyte concentration measurement system according to the invention, with a displacement member in a partially inserted position;
Fig 7b is a view similar to figure 7a except that the displacement member is in a partially retracted position;
Figs. 8a - 8d are simplified schematic views of a body access unit of an analyte concentration measurement system according to the invention illustrating steps of operation of the system according to a first measurement method embodiment;
Figs. 9a - 9d are simplified schematic views of a body access unit of an analyte concentration measurement system according to the invention illustrating steps of operation of the system according to a second measurement method embodiment;
Figs. 10a - 10d are simplified schematic views of a body access unit of an analyte concentration measurement system according to the invention illustrating steps of operation of the system according to a third measurement method embodiment;
Fig 11 shows a simplified cross section of another embodiment of a body access unit of an analyte concentration measurement system according to the invention;
Fig 12 shows a simplified cross section of another embodiment of a body access unit of an analyte concentration measurement system according to the invention.

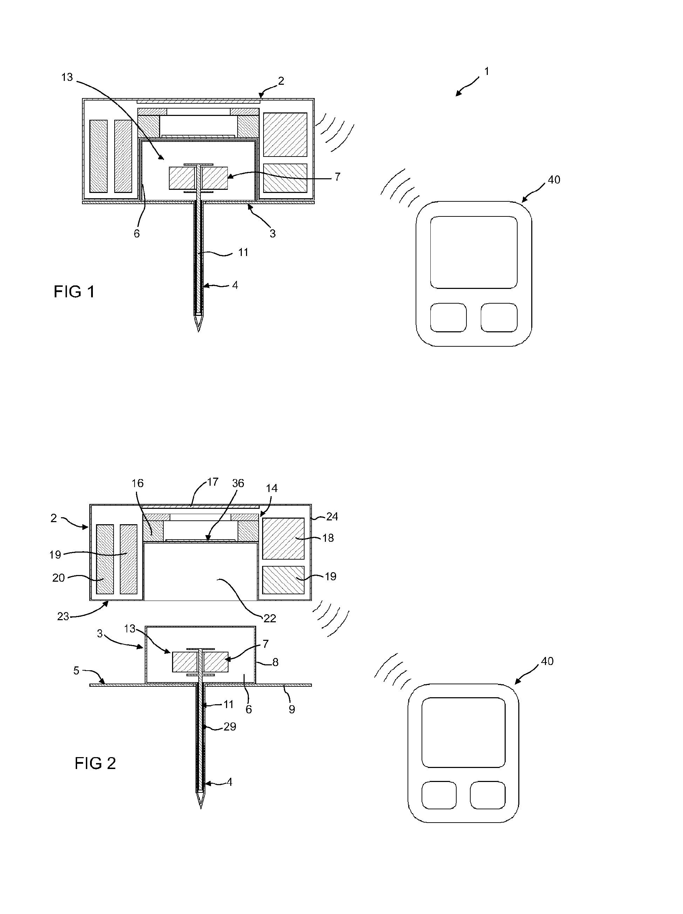

Referring to figures 1 and 2, an embodiment of an analyte concentration measurement system according to the present invention is illustrated. The analyte concentration measurement system 1 comprises a body access unit 3, a processing unit 2, and optionally a separate user interface 40.

Figure 1 shows the processing unit 2 attached to the body access unit 3. Figure 2 shows the processing unit 2 detached from the body access unit 3.

In a preferred embodiment, the two units 2 and 3 are detachably attached to each other, or separably mounted one against the other, when an analyte concentration is to be measured by the system. In a preferred embodiment, the body access unit 3 is disposable, whereas the processing unit 2 is reusable. This arrangement allows for a cost effective and ecological application of the system, as the more valuable parts contained in the processing unit 2 are used over a longer period of time than the consumable parts contained in the body access unit 3.

The body access unit 3 and processing unit 2 could however be integrated to form a single inseparable unit that is configured to be applied to a patient. The functions of a user interface device could be integrated in the processing unit 2 or provided in the separate user interface 40.

Referring to figures 1 and 2, the processing unit 2 comprises a housing 24, an excitation means 14, a power source 20, and a signal processing section comprising a microprocessor 18. The processing unit may further comprise a user interface 17 including input and/or output means such as a display, buttons, indicating lamps or acoustic means. The signal processing section may further comprise a memory 19 and a communications module 21 for wired or wireless communication with the separate user interface 40 or an external computing device. The housing 24 of the processing unit provides a hermetic or waterproof enclosure of the electrical and electronic components of the processing unit. The processing unit housing 24 is preferably designed with a low height to allow for maximum wearing comfort, as the medical device is adapted to be used in daily life beneath the clothes of a user.

The body access unit 3 comprises a support member 5 such as an infusion set or base plate, a housing 8, a fluid reservoir 6 in the housing containing an analyte sensitive fluid, a transcutaneous dialysis member 4, and a movable displacement member 13. The displacement member comprises a drive portion 7, and an extension 11 inserted at least partially in a cavity 29 of the dialysis member 4. The fluid reservoir 6 thus extends from the housing 8 into the cavity 29 of the dialysis member 4. The dialysis member comprises an analyte porous membrane 12 that allows exchange of analyte molecule between the sensitive fluid and the body tissue and fluid surrounding the dialysis member, as will be explained in more detail further on.

The support member 5 is fixed to the assembly of the transcutaneous dialysis member 4 and housing 8, and may for example form a patch configured for mounting against a patient's skin.

The support member 5 preferably comprises a lower support member surface 9 which is adapted to adhere to the skin of a patient. The support member may be provided in the form of a patch, as shown in figures 1 and 2.

In a preferred embodiment, as shown in figures 1 and 2, the support member 5 in the form of a patch covers substantially the same surface area as the processing unit 2. This enhances wearing comfort, and no part of the processing unit is in direct contact with the skin of the user, such that a long term use of the processing unit is not limited by hygiene constraints. Alternatively, if the area of the mounting surface 23 of the processing unit is larger than the surface area of the support member 5, hygiene problems may be prevented by using disposable adhesive patches (not shown) between the skin of a user and the mounting surface of the processing unit.

The body access unit 3 as a whole is disposable and its components are preferably non-detachably fixed to each other to ensure easy manipulation by a user.

In the separable body access unit 3 and processing unit 2 embodiment, the processing unit housing 24 is preferably provided with an interface docking cavity 22 on one side thereof to enable the excitation means to be placed against and around the housing 8 and displacement member 13 of the body access unit 3. This embodiment is especially useful when magnetic excitation means are used, in order to reduce energy requirements.

The functional connection between the processing unit 2 and the body access unit 3 is preferably achieved by magnetic or electromagnetic fields to avoid a direct electrical connection between the two units.

To attach the processing unit 2 to the body access unit 3 during analyte concentration measurement, coupling means (not shown) may be provided. For instance, the lower housing surface 23 of the processing unit may be provided with a magnet (not shown) or a magnetic material attracted to a magnetic material respectively magnet (not shown) provided in a support member 5 or housing 8 of the processing unit. Alternatively, mechanical coupling means (not shown) may be provided on the processing unit 2 and on the body access unit 3 in order to secure the two units together in a situation of use. Preferably, the securing mechanism allows repeatedly attaching and detaching of the two units, ensuring maximum flexibility and freedom when using the system.

A displacement sensor 36 may be provided in the processing unit 2 to measure the displacement behavior of the displacement member 13, and may be in the form of a capacitive sensor, or any other suitable sensor such as a Hall sensor, or an optical sensor such as a laser sensor. In another variant, the sensor may be integrated in the regulation circuit of the excitation means in the processing unit. For example, the displacement behavior sensor may include a force or position sensing function integrated in the control circuit of an electromagnetic drive forming the excitation means, the electromagnetic drive (e.g. coils) acting upon a permanent magnet of the displacement member 13. In other terms, the drive in the processing unit 2 and the permanent magnet on the displacement member in the body access unit form a motor that is controlled by the control circuit, whereby the control circuit may be provided with means to determine the position of the permanent magnet and/or the electro-motive force acting thereon, e.g., by measuring the current flowing through the electromagnetic coils.

The signal processing circuit comprising a microprocessor 18 and a memory 19 is provided to process the measurement signals, in particular the displacement behavior of the displacement member, into a value representing the analyte concentration. Said value may be displayed and or recorded on the processing unit 2, or sent to the remote user interface device 40.

A communication module 21 may be provided in the process unit housing 24 for sending and or receiving measurement data and or instructions.

A power source 20 is preferably provided in the processing unit 2. This has the advantage that one power source may be used with multiple body access units 3, which allows ecologic and economic use of the analyte measurement system. A separate power source may also be included in the body access unit 3. In an alternative embodiment (not shown), a power source may be included in the body access unit 3 together with electrical contacts connecting the power source with the electronic components in the processing unit. The advantage of the latter variant is that the processing unit 2 does not require a power source since each new body access unit 3 provides a full-capacity power unit, and it is therefore not necessary to replace or to recharge a power source in the processing unit.

The processing unit 2 may further comprise an alarm unit (not shown) to inform the user if the measured analyte concentration lies outside a predefined range, or if the medical device is subject to malfunction, or if the body access unit 3 is not correctly attached to the processing unit 2. The alarm may comprise visual, acoustic, vibratory or any other suitable means to attract the attention of the patient.

The transcutaneous dialysis member 4 is inserted into the patient skin, such that the dialysis member 4 is located at least partially in corporeal fluid (preferably interstitial fluid or blood) of the user. The communication module 21 comprised in the process unit 2 communicates wirelessly with a user interface device 40, which may be worn as a wrist watch. In figure 2, the processing unit 2 is detached from the body access unit 3. In this condition, the analyte concentration measurement system is not functionally connected and no measurement of the analyte concentration can be performed. However, if the user e.g. takes a shower or otherwise exposes the medical device to a hazard, the processing unit 2 can easily be detached from the body access unit 3 in order to conserve the more valuable processing unit 2. As soon as measurement of the analyte concentration needs to be carried out, the processing unit 2 can be re-attached to the body access unit 3. Alternatively, the processing unit 3 can be fully sealed and in effect be rendered water-proof, such that it is unnecessary for the user to disconnect the processing unit 3 from the body access unit 2.

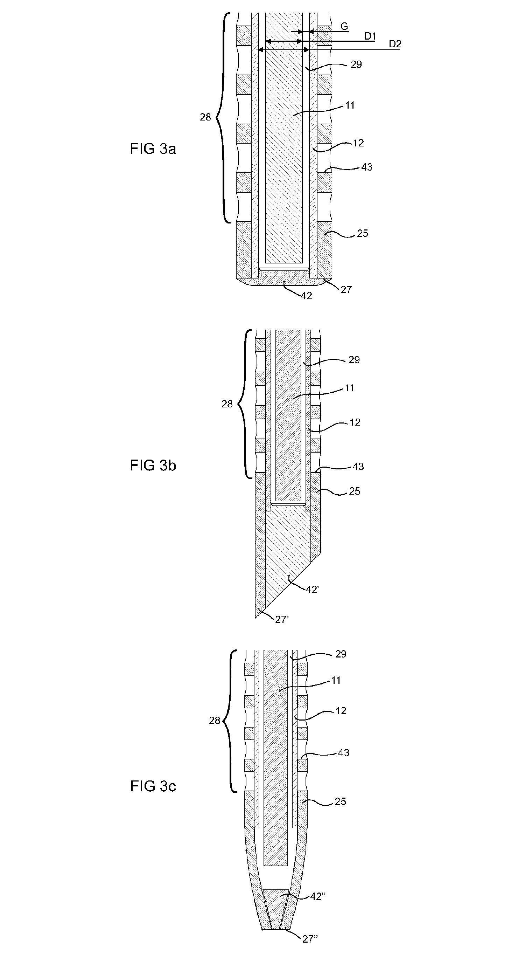

Referring to figures 3a to 4b, the dialysis member 4 comprises a support tube 25 with orifices 43 along an analyte exchange section 28 thereof, an analyte porous membrane 12 adapted to allow selective exchange of molecules between the sensitive fluid in the dialysis member cavity 29 and the patient's body fluid surrounding the dialysis member 4. Preferably, the transcutaneous dialysis member is provided in a substantially rigid form, e.g. in the form of a needle, which may also perform the function of perforation and insertion into the patient's tissue. In the latter embodiment, the support tube 25 may for example be made of a steel tube similar to medical tubes used for syringe needles. The transcutaneous dialysis member could however be provided with other shapes and forms, and be elastic or flexible, and moreover could be inserted transcutaneously by separate perforating means. The support tube could in this case be made for example of a polymer material with the desired stiffness or flexibility.

The support tube 25 provides a mechanical support for the porous membrane 12, and may also include a perforating tip 27', 27" (Fig. 3b, Fig. 3c) to facilitate insertion of the dialysis member through the skin of a user. The tip of the support tube may be sealed by a plug 42, 42', 42" of resin, glue or other material with suitable biological properties. The perforating tip 27" as shown in fig. 3c may be formed by deformation (e.g. crimping) the end on the support tube, or as shown in fig. 3b (tip 27') by conventional needle bevel tip forming techniques.

The analyte exchange section 28 is preferably located such that in a situation of use this section is surrounded by interstitial fluid. As the interstitial fluid is found below the skin surface, the analyte exchange section preferably is located near the dialysis member tip 27, 27', 27".

The analyte porous membrane 12 comprises pores of such a size that analyte molecules, e.g. glucose, can pass through the pores, whereas larger molecules found in the bodily fluids, such as proteins, are prevented from passing. In an embodiment, the porous membrane may comprise a tubular regenerated cellulose or cellulose ester fiber. The pore radius is preferably in the 1-10nm range, most preferably in the 2-4nm range.

The displacement member extension 11 has a diameter D1 slightly smaller than the inner diameter D2 of the analyte porous membrane 12, forming a fluid flow gap G. As the displacement member extension 11 is displaced during measurement, analyte sensitive liquid is forced through the fluid flow gap G. As the analyte sensitive liquid changes its viscosity depending on the analyte concentration, the liquid flow force acting on the displacement member 13 is a measure for the analyte concentration present in the analyte sensitive liquid.

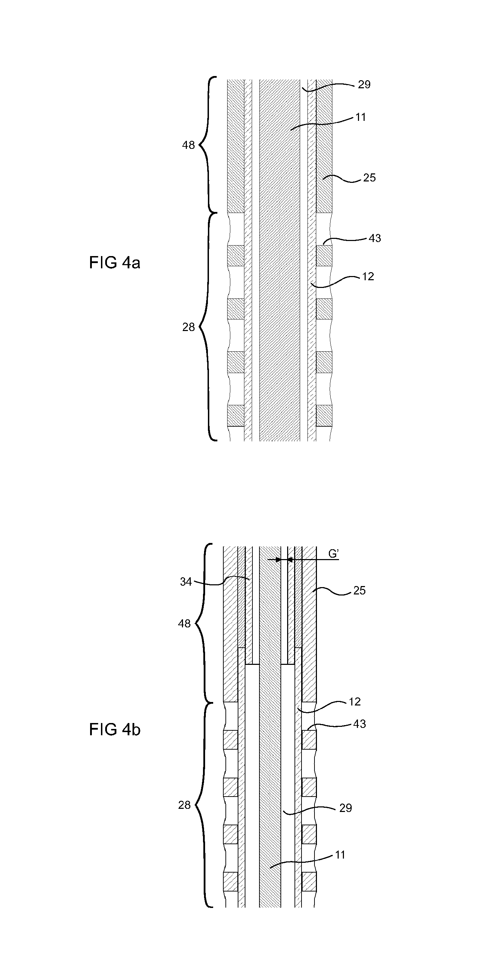

As shown in Fig. 4b, if an increased volume of sensitive fluid in the analyte exchange section 28 is required or desired, a constriction in the form of a tube 34 or annular protuberance may be mounted in the cavity 29 of the dialysis member in the analyte measurement section 48, above the analyte exchange section 28. The constriction 34 enables optimization of the desired gap G' between the extension 11 and the cavity inner diameter to optimize the flow resistance, while allowing a sufficient supply volume of analyte containing sensitive fluid to be available for effective viscosity measurement over displacement stroke of the extension 11.

The outer diameter of the transcutaneous dialysis member 4 may be between 0.1 and 0.5 mm, preferably between 0.25 and 0.35 mm for optimum patient comfort and device manufacturability. The length of the transcutaneous dialysis member implanted through the patient's skin may be between 2 and 10 mm, most preferably between 3 and 6 mm.

The fluid reservoir 6 preferably contains a volume of sensitive liquid much larger than the volume in the dialysis cavity 29, preferably at least 100 times bigger than the volume contained in the transcutaneous dialysis member 4, but preferably greater than 1000 times. The volume of the fluid reservoir 6 serves as a reservoir of new analyte sensitive liquid, such that for each new measurement new analyte sensitive liquid from the housing fluid reservoir 6 flows into the dialysis cavity 29. The variation in analyte concentration in the reservoir due to fluid expelled from the cavity 29 of the dialysis member 4 is negligible over the lifetime of the disposable body access unit in view of the relative volume. The variation in analyte concentration in the fluid reservoir 6 can be further reduced by providing a sensitive fluid in the reservoir 6 containing analyte molecules at a specified concentration, for example at a concentration close to an average concentration of analyte expected in the body fluid to be measured, so that the circulation of fluid from the dialysis cavity 29 into the reservoir 6 does not change the analyte concentration in the reservoir 6 in any measurable or significant amount. In other words, in a variant, the analyte sensitive liquid may contain a concentration of the analyte to be measured corresponding essentially to an analyte concentration at an average physiological concentration such that deviations of the analyte concentration in the body essentially occur around the mean analyte concentration in the analyte sensitive liquid.

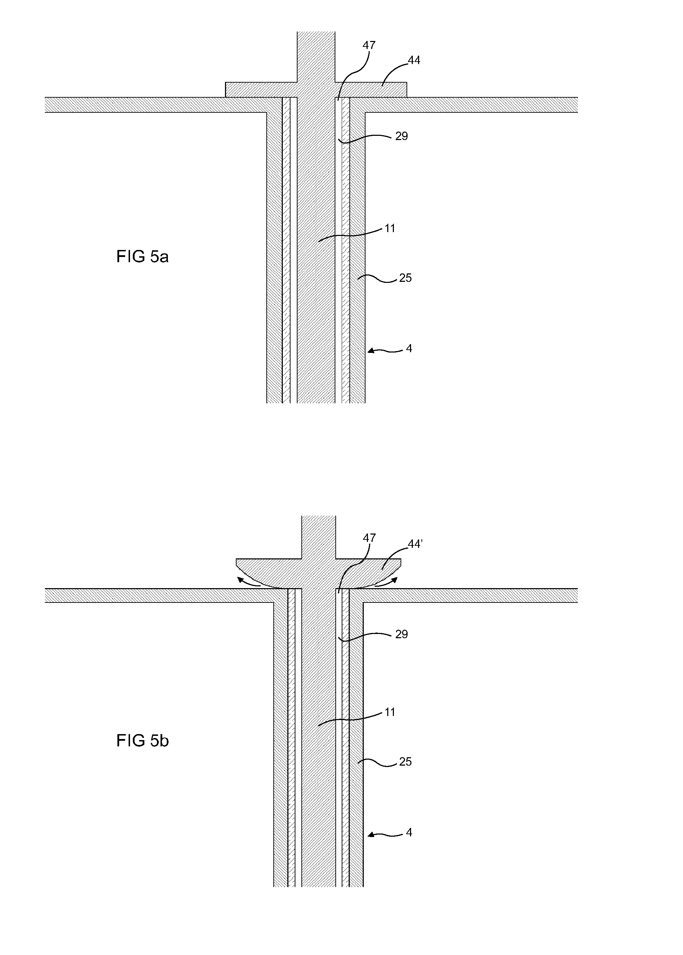

Referring to figures 5a and 5b, the extension 11 of the displacement member 13 may comprise a stopper 44, 44' that abuts against the inlet 47 of the cavity 29 at the end of the displacement member insertion stroke. The stopper 44, 44' expels fluid radially outwards towards the end of the insertion stroke to increase mixing of the expelled fluid with fluid in the reservoir 6. This is advantageous to avoid analyte concentration gradients in the reservoir 6 close to the inlet 47, in view of preparing for the return stroke of the displacement member whereby new fluid is sucked back into the cavity 29. The shape of the stopper 44, 44' - for example flat as in fig. 5a or convex as in figure 5b - can be optimized to expel liquid efficiently and favor mixing, depending on various parameters such as the fluid viscosity and fluid flow rate, and cavity dimensions.

The analyte sensitive liquid can be selectively adapted to the analyte to be measured, e.g. a receptor protein selectively binding the analyte may be contained in the analyte sensitive liquid, whereas the viscosity depends on the concentration of analyte molecules bound by the protein. In the case that the analyte is glucose, the analyte sensitive liquid is a glucose sensitive fluid such as a mixture containing concanavalin A and dextran or phenylboronic acids. Such glucose sensitive liquids are *per se* well known in the art and shall not be discussed in further detail.

Referring to figures 1, 2, the excitation means 14 comprises, in a preferred embodiment, an electromagnetic stator comprising one or more coils 16 and possibly one or more permanent magnets (not shown) to drive in translation or in translation and rotation (depending on the embodiment) the displacement member 13 of the body access unit 3. Permanent magnets are advantageous as they are known in the art to generate a stable and reproducible magnetic field, as required for a reliable and accurate displacement behavior of the displacement member. It may thus be advantageous to apply an electromagnetic force by means of the coils 16 on the displacement member 13 only for displacing in one direction, e.g., for retraction, respectively for insertion, while letting the displacement member return to its equilibrium inserted, respectively retracted position by means of the magnetic force exerted by the permanent magnets. The displacement member 13 comprises a drive portion 7, in an embodiment in the form of or comprising a permanent magnet 7, driven by the electromotive force generated by the one or more coils 16 of the electromagnetic stator. The drive portion 7 may comprise one or more magnets or one magnet with one or more magnetic segments formed by N-S pairs of opposite magnetic polarity. Also, the drive portion may comprise a soft iron support structure or body to configure the magnetic circuit between the mobile component and the static component of the motor. Various configurations of magnets and soft iron magnetic cores are possible, for example as found in conventional linear or rotational electromagnetic motors.

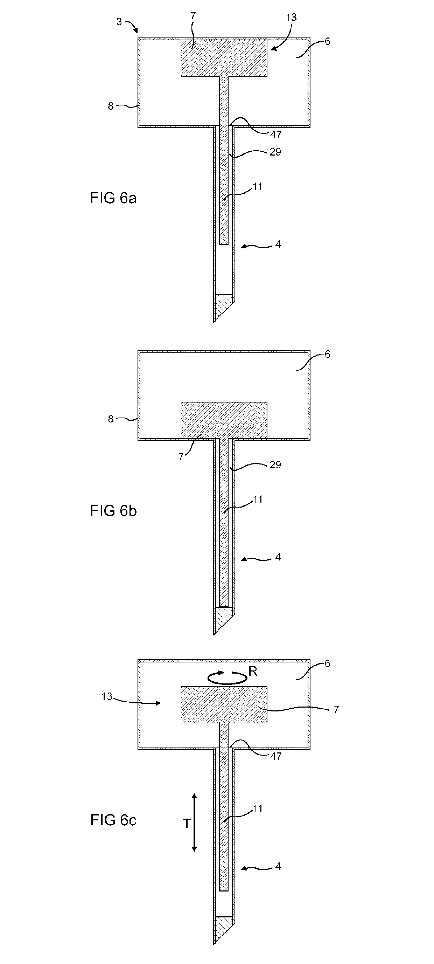

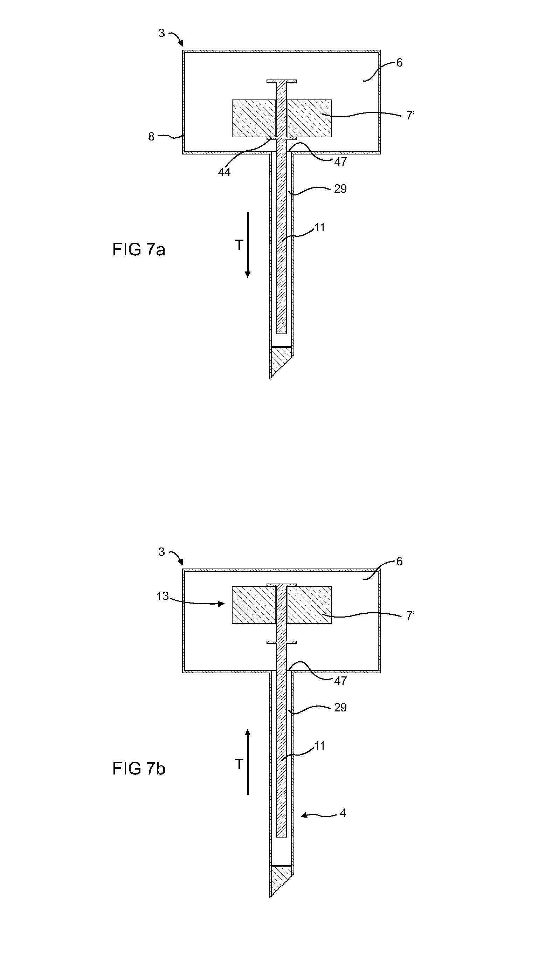

The drive portion 7 of the displacement member may be integrally or immovably fixed to the extension as illustrated in the embodiment of figures 6a to 6c, or movably mounted 7' to the extension 11 as illustrated in the embodiment of figures 7a, 7b. In the latter embodiment, the drive portion 7' comprises a magnet that is slidably and optionally rotatably mounted on the extension 11 to allow movement in the translation direction T, optionally in rotation R, for the purpose of improving mixing of the fluid in the reservoir, particularly the fluid expelled from the cavity 29 of the dialysis member in the region of the connection 47 between the reservoir and the cavity 29.

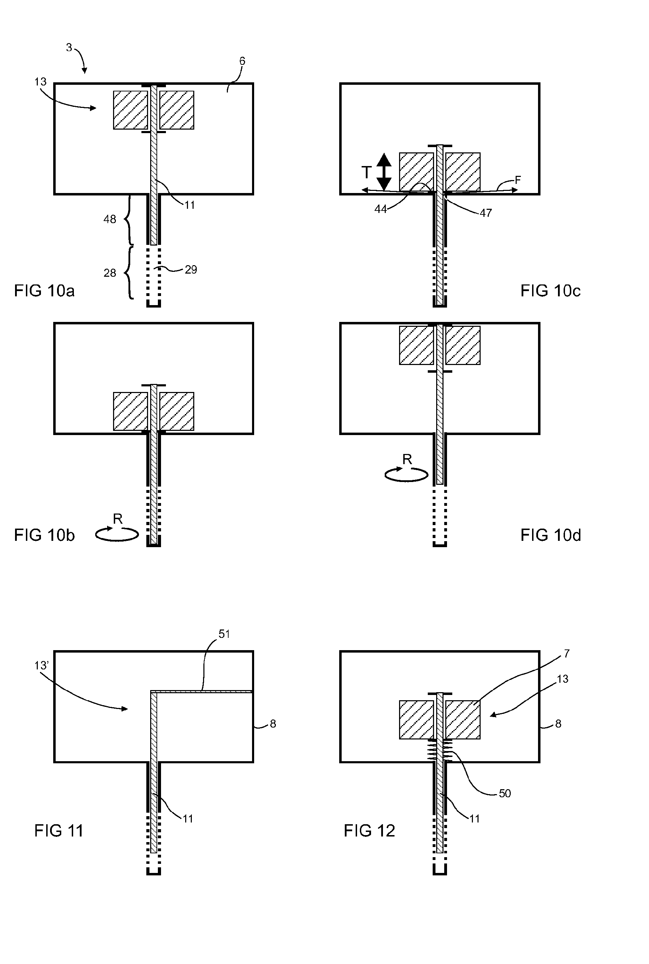

As illustrated in Figure 12, the displacement member 13 may be biased in a stable retracted or in an inserted position by spring means 50 arranged between the displacement member and the housing 8. The biasing means ensure that the displacement member is in a stable position ready for the measurement cycle in the absence of power, whereby the actuation of the displacement member acts against the biasing means to displace the member 13 during the measurement cycle. The biasing function may also be provided by a permanent magnet mounted to the housing attracting the displacement member to the stable position.

As illustrated in Figure 11, in a variant the displacement member 13 may be coupled to the housing 8 by a mechanical actuation member 51 such as a beam, for instance an elastic cantilever, or other physical coupling member that may be actuated to displace the displacement member. The mechanical actuation member may for instance be actuated by a piezoelectric element, a capacitive element or other force generators acting on the cantilever 51 or on the displacement member 13'. The actuation member may also include a displacement sensor to measure the displacement behavior of the displacement member 13'. The mechanical actuation member 51 may also perform the function of maintaining the displacement member 13' in a stable position ready for the measurement cycle in the absence of power.

Referring to figure 6a to 7b, embodiments of measurement processes shall now be described.

In a preferred embodiment, the movement of the displacement member 13 comprises a translational movement T from a retracted position as illustrated in fig. 6a to an inserted position as illustrated in fig. 6b, such that the extension 11 is inserted further into the cavity 29 of the transcutaneous dialysis member 4 thus displacing analyte sensitive liquid out of said cavity into the fluid reservoir 6. The displacement member 13 then performs a return translational movement retracting the extension 11 such that analyte sensitive fluid from the fluid reservoir 6 enters the cavity 29. The actuation of the displacement member in this variant is a linear oscillation or oscillatory displacement. It should be noted that the terms "oscillation" or "oscillatory displacement" are meant herein to encompass a displacement that may have more than one cycle or that could be less than a full oscillation cycle, for example the displacement member 13 may be driven in only one direction, e.g., preferably by means of the electromagnetic coils 16 and then released, whereby the return displacement behavior of the displacement member 13 into its original position e.g., preferably under action of permanent magnets (not shown) in the processing unit 3, is measured. The oscillatory behavior of the displacement member depends on the dimensions of the components on the one hand, and on the resistance caused by the analyte sensitive liquid in the fluid reservoir 6 and in the cavity 29 of the transcutaneous dialysis member 4. The damping effect of the analyte sensitive liquid on the oscillation depends *inter alia* on the viscosity of the analyte sensitive liquid, which, in the exchange section 28 of the transcutaneous dialysis member, varies with the concentration of analyte. With fluidic computations of the displacement of the displacement member 13, it is possible to define the gap G, G', such that the contribution of the drive part 7 of the displacement member 13 to the resistance to displacement is negligibly small compared to the contribution of the extension 11.

In an alternative embodiment, the extension 11 of the displacement member 13 may comprise blades or a helical thread (not shown) or equivalent fluid pumping means there along and the movement of the displacement member 13 may comprise a rotational movement such that, as the extension 11 rotates inside the cavity 29 of the transcutaneous dialysis member 4, analyte sensitive liquid is circulated out of said cavity 29 into the fluid reservoir 6 and from the fluid reservoir 6 into the cavity 29.

In a further alternative embodiment, the displacement member 13 may be configured to move both in a translational movement T and a rotational movement R in order to pump fluid out of, respectively into the cavity 29 of the dialysis member 4, and to mix the fluid in the fluid reservoir 6, at least in the vicinity of the connection between the cavity 29 of the dialysis member 4 and the fluid reservoir 6.

In the initial displacement of the displacement member 13, the analyte sensitive fluid expelled from the dialysis member cavity 29 has a viscosity that is dependant on the concentration of analyte in fluid in the exchange section 28 of the cavity 29 (hereinafter referred to as the analyte infused sensitive fluid), which is dependant on the concentration of analyte in the external fluid (i.e. body fluid) surrounding the dialysis member 4 in view of the exchange of analyte molecules through the analyte porous membrane 12. The flow of analyte sensitive fluid expelled from the dialysis member cavity 29 to the fluid reservoir is restricted by the resistance acting on the dialysis member extension 11 inserted in the cavity 29 that is dependent on the viscosity of the fluid. The displacement behavior of the displacement member thus depends on the fluid flow resistance acting on the displacement member which in turn depends at least partially on the viscosity of the analyte sensitive liquid flowing in and out of the dialysis member cavity 29. During the initial movement of the displacement member 13, the fluid in the exchange section 28 of the dialysis member cavity 29 has an analyte concentration that corresponds to the analyte concentration on the outer side of the porous membrane 12 and thus has a viscosity correlated to the external analyte concentration. After the initial movement expelling fluid from the cavity 29 of the dialysis member 4 and subsequent refilling of the cavity section with fresh fluid from the reservoir, the viscosity of the fluid changes, whereby after a few cycles of displacement of the displacement member 13, the fluid in the cavity section 29 of the dialysis member 4 is to a large extent refreshed and has a viscosity corresponding essentially to the viscosity of the analyte sensitive fluid in the reservoir 6 that is not correlated to the external analyte concentration and thus serves as a reference.

The behavior of the displacement of the displacement member 13 in the initial movement or movement cycle can be compared to the displacement behavior of the displacement member 13 in one or more subsequent movements or movement cycles thereby allowing calibration of the viscosity of the analyte infused sensitive fluid with the viscosity of the reference sensitive fluid in the fluid reservoir 6.

Advantageously, this relative viscosity measurement eliminates or reduces the requirements for regular external calibration of the medical device measurement parameters compared to a system based on measuring an absolute viscosity. The relative increase in viscosity is well correlated to the absolute analyte concentration in the analyte infused sensitive fluid and is little affected by the temperature or ageing of the sensitive fluid.

The detail steps in the measurement process relying on the above described measurement principle may vary however according to various embodiments, examples of which are presented below.

### Examples of measurement process variants according to the invention:

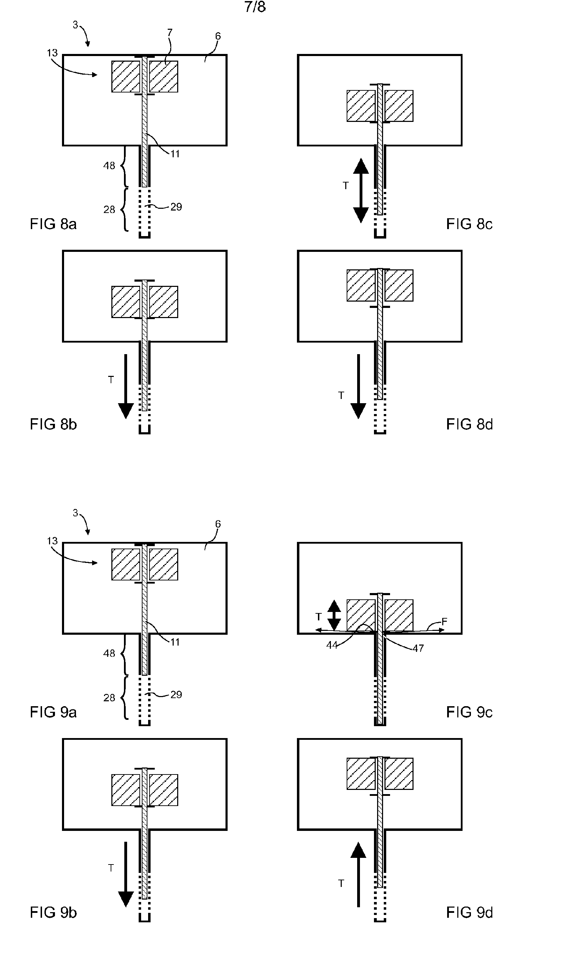

### I. Embodiment #1 (illustrated by figures 8a-8d)

Viscosity (both measurement of analyte infused sensitive fluid and reference fluid from reservoir) is measured by a downwards movement of the displacement member.
Step 1 (Fig. 8a): analyte-exchange mode
   - Displacement member 13 is fully retracted
Step 2 (Fig. 8b): measurement mode
   - Displacement member 13 is inserted in the cavity 29 of the dialysis member 4
   - Fluid is expelled from exchange section 28 through viscosity measurement section 48
   - Resistance to flow dependant on viscosity, of fluid in the measurement section 48 relate to analyte concentration in the body
Step 3 (Fig. 8c): homogenization mode
   - Displacement member 13 is inserted and retracted several times until the analyte concentration in the dialysis member 4 is essentially identical to that in the reservoir 6
Step 4 (Fig. 8d) : calibration mode
   - Displacement member 13 is inserted in the cavity 29 of the dialysis member 4
   - Fluid is expelled from the exchange section 28
   - Resistance to flow dependant on viscosity, of fluid in the measurement section 48 relate to reference analyte concentration in the reservoir

### Benefits of Embodiment #1:

1. System always measures in the same displacement member direction. With the use of permanent magnets in the processing unit 2, the displacement member movement can be well-controlled. The inserting, measuring, displacement of the displacement member 13 may thus be provided by permanent magnets, while the retracting, mixing, displacement of the displacement member 13 may be provided by electromagnetic coils 16.
2. The displacement member 13 always moves downwards for the measurement, so that it is done with an overpressure. The upwards movement can be slower by means of electromagnetic coils to prevent air bubbles formation.

### II. Embodiment #2: (illustrated by figures 9a-9d)

Viscosity of analyte infused sensitive fluid is measured by a downwards movement of the displacement member 13, immediately followed by a calibration using an upward movement of the displacement member 13
Step 1 (Fig. 9a): analyte-exchange mode
   - Displacement member 13 is fully retracted
Step 2 (Fig. 9b): measurement mode
   - Displacement member 13 is inserted in the cavity 29 of the dialysis member 4
   - Fluid is expelled from exchange section 28 through viscosity measurement section 48
   - Resistance to flow/ Viscosity of fluid in the measurement section 48 relate to analyte concentration in the body
Step 3 (Fig. 9c): Homogenization mode
   - Displacement member is fully inserted
   - Lower magnet stopper 44 is designed to efficiently expel (F) liquid from the cavity 29 in the reservoir 6
   - (Optional) magnet oscillation homogenizes fluid in the reservoir 6, for instance by generating an alternating magnetic force by means of the electromagnetic coils
   - After a few cycles, sensitive fluid close to stopper 44 is homogenized to the analyte concentration in the reservoir 6
Step 4 (Fig. 9d): calibration mode
   - Displacement member 13 is retracted
   - Fluid from the reservoir 6 is pulled into the measurement 48 and exchange 28 sections
   - Resistance to flow dependant on viscosity, of fluid in the measurement section 48 relate to reference analyte concentration in the reservoir

### Benefits of Embodiment #2:

1. Reference measurement relies directly on the viscosity of liquid from the reservoir, instead of liquid in the needle. It is therefore not subject to interferences from analyte diffusion through the porous membrane 12 during the homogenization step.
2. The homogenization takes place in the reservoir and the needle remains stationary during this step.

### III. Embodiment #3 (illustrated by figures 10a-10d)

Analyte concentration viscosity is measured by a rotational movement of the displacement member, followed by a homogenization by means of a translational movement and another rotational measurement
Step 1 (Fig. 10a): analyte-exchange mode
   - Displacement member 13 is fully retracted
Step 2 (Fig. 10b): measurement mode
   - Displacement member 13 is inserted in the cavity 29 of the dialysis member 4
   - Fluid is expelled from the exchange section 28
   - Viscosity of fluid in measurement section 48 and exchange section 28 relates to interstitial analyte concentration
   - Viscosity is measured by means of a rotational movement of the displacement member 13
Step 3 (Fig. 10c): Homogenization mode
   - Displacement member is fully inserted
   - Lower magnet stopper 44 is designed to efficiently expel (F) liquid from the dialysis member cavity 29 into the reservoir 6
   - (Optional) magnet oscillation homogenizes fluid in the reservoir 6, for instance by generating an alternating magnetic force by means of the electromagnetic coils
   - After a few cycles, sensitive fluid close to stopper 44 is homogenized to the analyte concentration in the reservoir 6
      Note: the homogenization mode of Embodiment #1 can also be used.
Step 4 (Fig. 10d): calibration mode
   - Displacement member 13 is retracted
   - Fluid from the reservoir 6 is pulled into the measurement 48 and exchange 28 sections
   - Resistance to flow dependant on viscosity, of fluid in the measurement section 48 relates to reference analyte concentration in the reservoir
   - The viscosity is measured by means of a rotational movement of the displacement member 13

### Benefits of Embodiment #3:

1. Reference measurement relies directly on the viscosity of liquid from the reservoir, instead of liquid in the needle. It is therefore not subject to interferences from analyte diffusion through the porous membrane 12 during the homogenization step
2. The linear movement of the displacement member does not have any metrological function, which loosens specifications on the translational drive.
   Both measurement and calibration are performed relying on the same (rotational) displacement member movement and drive while alleviating the risk of interference of analyte diffusion during the mixing step.

## Claims

1. Analyte concentration measurement system comprising a body access unit (3), the body access unit including:
a transcutaneous or implantable dialysis member (4) comprising an analyte porous membrane (12) disposed at least along an analyte exchange section (28) of said dialysis member,
a fluid reservoir (6) extending connected to a cavity (29) of the dialysis member, the cavity bounded at least partially by the analyte porous membrane,
an analyte sensitive liquid contained in the fluid reservoir and dialysis member cavity, and
a displacement member (13) configured to be displaced in a predefined manner, the displacement of the displacement member inducing flow of the analyte sensitive liquid in the cavity of the dialysis member,
wherein the displacement member comprises an extension (11) inserted in the cavity of the dialysis member and configured to displace analyte sensitive liquid out of the analyte exchange section (28).

2. Analyte concentration measurement system according to claim 1 wherein the dialysis member is configured for transcutaneous implantation in a patient and for insertion of the analyte exchange section in body fluid, whereas the fluid reservoir (6) is configured for extracorporeal disposition.

3. Analyte concentration measurement system according to claim 2 wherein the dialysis member is rigid and has a perforating tip (27', 27") configured to perforate tissue.

4. Analyte concentration measurement system according to any one of the preceding claims wherein the dialysis member comprises a perforated or porous support tube (25) around or within which the analyte porous membrane (12) is mounted.

5. Analyte concentration measurement system according to any one of the preceding claims wherein the body access unit comprises a support member (5) in the form of a patch configured to adhere to a skin of a user.

6. Analyte concentration measurement system according to any one of the preceding claims wherein the displacement member comprises a drive portion (7) positioned in the fluid reservoir (6), the drive portion comprising a permanent magnet.

7. Analyte concentration measurement system according to claim 6 wherein the drive portion (7') is movably mounted to the displacement member extension (11).

8. Analyte concentration measurement system according to the previous claim
wherein the drive portion is configured to be located extra corporeal and the displacement member extension (11) is configured to be located partially extra corporeal and partially intra corporeal in a situation of use.

9. Analyte concentration measurement system according to any one of the preceding claims wherein the analyte is glucose and the analyte porous membrane comprises a cellulose fiber.

10. Analyte concentration measurement system according to any one of the preceding claims further comprising an extra corporeal processing unit (2) including:
an excitation means (14) configured to drive the displacement member in movement to generate a flow of the analyte sensitive liquid contained in the dialysis member (4),
means to measure the displacement behavior of the displacement member; and
a signal processing unit configured to process the displacement behavior signal of the displacement member into a value representative of analyte concentration in the analyte sensitive liquid in the dialysis member.

11. Analyte concentration measurement system according to claim 10 wherein the excitation means comprises an electromagnetic stator drive.

12. Analyte concentration measurement system according to any one of the preceding claims wherein the displacement member extension (11) comprises a needle shape body and the displacement member cavity (29) comprises an elongated tubular shape having a diameter (D2) greater than a diameter (D1) of the extension such that a fluid flow gap (G), effective for fluid viscosity measurement based on the displacement behavior of the extension, is formed therebetween.

13. Analyte concentration measurement system according to any one of the preceding claims wherein the displacement member extension (11) and dialysis cavity (29) are configured for translational displacement (T) of the extension in the cavity.

14. Analyte concentration measurement system according to any one of the claims 1-12 wherein the displacement member is configured to move both in a translational movement (T) and a rotational movement (R) in order to pump fluid out of, respectively into the cavity of the dialysis member, and to mix the fluid in the fluid reservoir, at least in the vicinity of the connection (47) between the cavity of the dialysis member and the fluid reservoir.

15. Analyte concentration measurement system according to any of the preceding claims, whereby the analyte sensitive liquid contains a concentration of the analyte essentially corresponding to an average physiological concentration such that deviations of the analyte concentration in the body essentially occur around the mean analyte concentration in the analyte sensitive liquid.

16. A method of operating the device according to any one of the preceding claims, including the steps of:
actuating the excitation means to move the displacement member, thereby expelling analyte sensitive liquid out of the cavity of the analyte exchange section of the dialysis member with the displacement member extension;
measuring the viscosity of the analyte sensitive liquid based on the displacement behavior of the displacement member.

17. Method of operating the device according to claim 16 wherein the movement of the displacement member (13) includes
a translational movement (T) from a retracted position to an inserted position where the extension (11) is inserted further into the cavity (29) of the dialysis member (4) thus displacing analyte sensitive liquid out of said cavity into the fluid reservoir 6, and
a return translational movement retracting the extension (11) such that analyte sensitive fluid from the fluid reservoir (6) enters the cavity (29).

18. A method of measuring an analyte concentration including the steps of:
providing a medical device including a body access unit comprising a transcutaneous dialysis member having an analyte exchange section with an analyte porous membrane, a fluid reservoir containing an analyte sensitive liquid, and a displacement member comprising an extension inserted in a cavity of the transcutaneous dialysis member, and a processing unit comprising an excitation means configured to displace the displacement means;
displacing the displacement member by means of the excitation means, thereby expelling analyte sensitive liquid out of the cavity of the analyte exchange section of the dialysis member with the displacement member extension;
measuring the displacement behavior of the displacement member and determining an analyte concentration correlated to the displacement behavior of the displacement member.

19. Method according to claim 18 wherein the analyte concentration measurement system comprises any of the further features of the analyte concentration measurement system according to any of the claims 1 to 13.

20. Method according to claims 17 or 18, whereby the return translational movement is used to provide a reference measurement.
